(19) Europäisches Patentamt
European Patent Office
Office européen des brevets

(11) Numéro de publication: **0 288 337 A1**

(12) # DEMANDE DE BREVET EUROPEEN

(21) Numéro de dépôt: **88400651.1**

(22) Date de dépôt: **18.03.88**

(51) Int. Cl.4: **C 07 B 41/00**
C 09 K 11/07
// C07D493/08, C07J71/00, C07C49/796, C07C49/784, C07D321/00, C07C179/025, C07C45/28, C07C178/00

(30) Priorité: **20.03.87 FR 8703891**

(43) Date de publication de la demande: **26.10.88 Bulletin 88/43**

(84) Etats contractants désignés: **AT BE CH DE ES FR GB GR IT LI LU NL SE**

(71) Demandeur: **UNIVERSITE DU DROIT ET DE LA SANTE (LILLE II)**
**42, rue Paul Duez**
**F-59800 Lille (FR)**

(72) Inventeur: **Aubry, Jean-Marie**
**13, rue Casimir Beugnet**
**F-62590 Oignies (FR)**

(74) Mandataire: **L'Helgoualch, Jean**
**OFFICE PICARD 134 Boulevard de Clichy**
**F-75018 Paris (FR)**

(54) **Procédé d'oxydation de composés organiques par l'eau oxygénée en présence d'un catalyseur.**

(57) L'invention concerne un procédé perfectionné d'oxydation de composés organiques.

Le procédé d'oxydation par l'eau oxygénée conforme à la présente invention, permettant d'oxyder des composés organiques en présence d'un catalyseur, se distingue en ce qu'on fait agir l'eau oxygénée sur le composé organique en présence d'un catalyseur constitué par un molybdate ou un tungstate de métal alcalin en microémulsion, obtenue par mélange d'une solution aqueuse du catalyseur, d'au moins un surfactif, et d'au moins un solvant organique.

Application à l'oxydation de composés organiques divers et à la chimiluminescence.

EP 0 288 337 A1

## Description

### Procédé d'oxydation de composés organiques par l'eau oxygénée en présence d'un catalyseur.

La présente invention concerne un procédé d'oxydation de composés organiques, et plus particulièrement un procédé d'oxydation par l'eau oxygénée en présence d'un catalyseur à base de sel de molybdène ou de tungstène.

On sait que la décomposition du peroxyde d'hydrogène (ou eau oxygénée) sous l'action de certains composés minéraux entraîne la formation d'oxygène singulet, forme excitée de l'oxygène ordinaire, qui réagit sur certains composés organiques pour former des peroxydes. Ainsi le système $H_2O_2/ClONa$ constitue un moyen d'oxydation efficace de divers composés tels que des oléfines, des dérivés aromatiques ou des cyanures, comme décrit dans les brevets US-A-3.274.181 et FR-A-2.321.455. Toutefois, un tel mélange eau oxygénée - eau de Javel, s'il permet de produire efficacement de l'oxygène singulet, présente l'inconvénient d'être trop agressif vis-à-vis de la plupart des composés à oxyder en raison de la grande réactivité de l'eau de Javel qui peut détruire la molécule à oxyder.

On connait également des méthodes photochimiques de production d'oxygène singulet, mais elles sont onéreuses et délicates à mettre en oeuvre industriellement. Elles ne peuvent donc convenir que pour de faibles quantités de produit.

On a donc été amené à rechercher d'autres sources d'oxygène singulet susceptibles d'être utilisables sur le plan industriel pour réaliser l'oxydation de divers composés. La formation d'oxygène singulet a ainsi été observée, dans certaines conditions, dans la décomposition du peroxyde d'hydrogène en présence d'un molybdate qui agit comme catalyseur (J.M. Aubry J. Am. Chem. Soc. Vol.107 (1985) p. 5844-5849). Cette réaction est avantageuse dans la mesure où sa mise en oeuvre est relativement aisée au laboratoire, et où le molybdate, en raison de sa fonction de catalyseur, peut être récupéré et recyclé. En outre, cette méthode est moins agressive que celle utilisant le mélange eau oxygénée - eau de Javel, et elle est alors applicable à l'oxydation de divers composés, et en particulier des molécules complexes et relativement fragiles dont on veut éviter la dégradation. Les essais effectués se sont cependant heurtés à des difficultés résultant notamment de problèmes de solubilité du molybdate et du composé à oxyder, et la méthode connue ne permet d'oxyder que des composés solubles dans l'eau ou dans un mélange eau/alcool.

Le brevet GB-A-1.324.763 décrit un procédé d'oxydation d'oléfines en phase hétérogène en présence d'un sel organique quaternaire et d'un métal, au moyen d'un oxydant choisi parmi l'acide paraperiodique, le peroxyde d'hydrogène et le permanganate de potassium.

La présente invention a pour objet un procédé perfectionné d'oxydation de composés organiques par l'eau oxygénée en présence d'un catalyseur, assurant une oxydation efficace tout en permettant une récupération totale du catalyseur, applicable industriellement à divers composés dans de bonnes conditions de rendement.

L'invention a également pour objet un procédé de production d'une chimiluminescence applicable comme source de lumière.

Le procédé d'oxydation par l'eau oxygénée conforme à la présente invention, permettant d'oxyder des composés organiques en présence d'un catalyseur, se distingue en ce qu'on fait agir l'eau oxygénée sur le composé organique en présence d'un catalyseur constitué par un molybdate ou un tungstate de métal alcalin en microémulsion.

Le pH du milieu réactionnel est compris entre 8 et 12,5 environ, et de préférence entre 10 et 11 environ. En dehors de ce domaine, la vitesse de décomposition de l'eau oxygénée est insuffisante. L'utilisation d'ions tungstate ou d'ions molybdate en solution aqueuse, conformément à la présente invention, présente l'avantage de procurer spontanément un pH compris entre 10 et 11 environ.

Le catalyseur utilisé dans l'invention est un tungstate ou un molybdate de métal alcalin, tel qu'un tungstate ou un molybdate de sodium ou de potassium. On utilise de préférence le molybdate de sodium ($Na_2MoO_4$), qui réagit plus rapidement que le tungstate de sodium ($Na_2WO_4$).

Le tungstate ou molybdate de métal alcalin est utilisé en solution aqueuse. La concentration doit être aussi élevée que possible dans la limite du domaine de stabilité de la microémulsion. Ainsi par exemple, la concentration en ions molybdate ou tungstate dans la phase aqueuse peut en général être comprise entre $10^{-3}$M et 1 M, et de préférence entre 0,1 et 0,4M. Une concentration plus élevée présente l'inconvénient d'entraîner une démixion de la microémulsion.

Conformément à l'invention, on prépare d'abord une microémulsion avant de faire agir l'eau oxygénée sur le composé organique à oxyder, en présence du catalyseur constitué par le tungstate ou le molybdate de métal alcalin.

La microémulsion est préparée en dissolvant le molybdate ou le tungstate de métal alcalin dans de l'eau, et en versant cette solution sous agitation dans un mélange d'au moins un solvant et d'au moins un surfactif pratiquement insoluble dans le solvant.

Il est avantageux que la phase aqueuse de tungstate ou de molybdate de métal alcalin contienne un tampon, et par exemple un tampon carbonate ou un tampon phosphate, capable de maintenir le pH à la valeur optimale pendant la réaction. Ainsi, à titre d'exemple, on peut utiliser notamment un mélange de carbonate de sodium et d'hydrogénocarbonate de sodium, qui permet d'éviter une diminution du pH du milieu réactionnel, et donc une diminution de la vitesse de réaction, au fur et à mesure de la disparition de l'eau oxygénée.

Le surfactif peut être choisi parmi les surfactifs anioniques usuels tels que les alkyl sulfates, les alkyléther

sulfates, les alkyl sulfonates, les alkylaryl sulfonates, les alkylamide sulfonates, les alkyl sulfosuccinates, et les alkyl phosphates de métal alcalin, comportant de préférence 10 à 18 atomes de carbone dans la chaîne alkyle. On peut utiliser par exemple le dodécyl sulfate de sodium, le dodécyl sulfate de potassium, ou le dodécyl benzène sulfonate de sodium. Le surfactif anionique peut être utilisé isolément ou en combinaison avec un ou plusieurs autres surfactifs.

Le surfactif anionique choisi doit exercer une interaction aussi faible que possible avec les ions présents dans le milieu réactionnel, c'est-à-dire les ions molybdate et permolybdate, ou les ions tungstate et pertungstate, de manière à ne pas perturber le processus catalytique. En outre, il importe de choisir un surfactif qui ne soit pas soluble dans le solvant organique utilisé pour faciliter le traitement ultérieur du milieu réactionnel.

Il est particulièrement avantageux d'utiliser un surfactif comme indiqué ci-dessus en combinaison avec un alcool inférieur suffisamment volatil pour pouvoir être facilement éliminé par évaporation après réaction. Cet alcool inférieur joue alors le rôle de co-surfactif. On peut utiliser, par exemple, le propanol-1, le butanol-1, le pentanol-1 ou l'hexanol-1.

Le solvant organique utilisé dans l'invention doit être un bon solvant des composés organiques à oxyder. Il est préférable que sa température d'ébullition soit relativement peu élevée, de telle sorte qu'il puisse être aisément éliminé après réaction de l'eau oxygénée. Il importe également que le solvant organique ne contienne pas d'impureté susceptible de modifier le pH de la microémulsion, et donc la vitesse de la réaction.

Le solvant organique peut être par exemple un hydrocarbure ou un hydrocarbure halogéné, tels que le dichlorométhane, le dichloro-1,2 éthane, le chloroforme, le tétrachlorure de carbone, le trifluoro-1,1,2 trichloro-2,2,1 éthane (Fréon 113), le n-pentane, le n-hexane, le cyclohexane, l'éther de pétrole, le benzène ou encore le toluène.

Comme indiqué ci-dessus, la microémulsion contient une solution de molybdate ou de tungstate de métal alcalin, au moins un surfactif, et au moins un solvant organique, ainsi que, le cas échéant, un tampon. On la prépare en dissolvant le molybdate ou le tungstate de métal alcalin dans de l'eau en présence du tampon carbonate, puis en versant cette solution dans une suspension contenant le surfactif, éventuellement le co-surfactif, et le solvant, et en maintenant l'ensemble sous agitation jusqu'à obtenir la microémulsion. Cette microémulsion est stable, et peut être utilisée immédiatement ou conservée pour une utilisation ultérieure.

La réaction d'oxydation s'effectue, conformément au procédé de l'invention, en faisant réagir l'eau oxygénée sur le composé organique à oxyder dans la microémulsion décrite ci-dessus. A cet effet, on peut par exemple dissoudre le composé organique dans la microémulsion dont le solvant a été choisi de manière appropriée, puis ajouter l'eau oxygénée. L'addition d'eau oxygénée peut s'effectuer en une seule fois, mais de préférence progressivement, et on peut par exemple opérer par additions successives ou en continu.

La température du milieu réactionnel, choisie en fonction du solvant et du composé à oxyder, est généralement comprise entre 10 et 80°C. Une température plus élevée risquerait de provoquer la décomposition des peroxydes formés, tandis qu'une température trop basse ne procure pas une vitesse de réaction suffisante. On peut faire varier la température dans le domaine indiqué ci-dessus de manière à obtenir une vitesse de réaction satisfaisante, sachant qu'une augmentation de température de 5°C entraîne une augmentation de 100% environ de la vitesse de formation d'oxygène singulet. On utilise de préférence une température de l'ordre de 20 à 40°C.

La réaction peut être suivie par chromatographie, par exemple par chromatographie liquide haute performance (CLHP). En fin de réaction, le solvant, l'eau, et l'alcool inférieur utilisé le cas échéant, peuvent être éliminés par évaporation sous vide, et le produit d'oxydation est récupéré par extraction et filtration.

L'eau oxygénée utilisée peut être celle disponible dans le commerce, par exemple une eau oxygénée à 30% (110 volumes). Il est avantageux d'utiliser une eau oxygénée de concentration plus élevée, par exemple à 50% ou 70%, qui présente l'avantage d'introduire moins d'eau dans le système pour une même quantité d'oxygène singulet formé, retardant ainsi la démixion de la microémulsion et permettant l'oxydation de composés organiques moins réactifs ou plus concentrés.

La concentration en eau oxygénée libre dans la phase aqueuse est de préférence comprise entre 0,1 et 1M pour optimiser la vitesse de formation d'oxygène singulet.

Le procédé conforme à la présente invention permet de réaliser l'oxydation de divers composés hydrophiles ou hydrophobes. Il est tout particulièrement avantageux pour l'oxydation de composés fortement hydrophobes qui ne peut être réalisée par les procédés chimiques connus. Il est notamment avantageux en synthèse organique où il permet de réaliser l'oxydation de composés organiques insaturés tels que des terpènes (par exemple l'α-terpinène et le citronellol), des dérivés de composés aromatiques polycycliques (par exemple le diphényl-9,10 anthracène et le rubrène), des stéroïdes (par exemple le benzoate d'ergostérol), des furannes (par exemple le diphényl-1,3 isobenzofuranne), des cyclopentadiènes (par exemple la tétracyclone), des phénols, etc, et plus généralement tous les composés connus pour réagir avec l'oxygène singulet.

Il permet également d'élaborer une source de lumière par réaction chimiluminescente, et présente donc de l'intérêt pour la mise au point d'éclairage transportable. On peut en effet, comme indiqué ci-après, utiliser comme composé organique à peroxyder, un composé insaturé conduisant à la formation de dérivé du type dioxétanne par réaction de cycloaddition [2+2] de l'oxygène singulet sur une double liaison ne comportant pas d'hydrogène allylique. La décomposition du dioxétanne en présence d'un fluoresceur provoque une émission de lumière. Il suffit donc d'introduire un tel composé organique, par exemple un dérivé de dioxène, et

un fluoresceur dans une microémulsion conforme à l'invention, et d'y ajouter de l'eau oxygénée. Dans ce cas, le surfactif utilisé pour la préparation de la microémulsion n'est pas nécessairement un surfactif anionique; il est également possible d'utiliser un surfactif non ionique tel qu'un alcool gras polyéthoxylé, qui permet de réduire les interactions ioniques.

Comme fluoresceur, on peut utiliser par exemple la rhodamine 6G, le diphényl-9,10 anthracène ou le bis(phényléthynyl)-9,10 anthracène.

Les exemples donnés ci-après illustrent l'invention plus en détail sans en limiter la portée.

Préparation d'une microémulsion

Dans un Erlenmeyer de 250ml, on introduit 7,8g de dodécylsulfate de sodium et 15,6g de butanol-1, puis on verse 100ml de dichlorométhane sous agitation. L'agitation est poursuivie pendant 5 minutes à température ambiante. On obtient ainsi une suspension à laquelle on ajoute une solution de 484mg de molyb date de sodium, 21mg de carbonate de sodium et 17mg d'hydrogénocarbonate de sodium dans 10ml d'eau.

L'agitation est poursuivie jusqu'à obtention d'une solution limpide. La solution obtenue est stable et peut être utilisée immédiatement ou ultérieurement. Il est cependant préférable de l'utiliser dans un délai d'une semaine car le dichlorométhane se décompose très lentement en dégageant de l'acide chlorhydrique qui diminue le pH de la solution.

La microémulsion ci-dessus convient tout particulièrement à l'oxydation d'un composé organique très hydrophobe. Dans le cas d'un composé hydrophile, tel que le citronellol, il suffit de diminuer la proportion de co-surfactif (butanol-1) et par exemple de n'en utiliser que 13,5g ou lieu de 15,6g.

**EXEMPLE 1**

Préparation de l'ascaridol

Dans un tricol de 500ml muni d'une ampoule à brome graduée, d'un refrigérant à reflux et d'un agitateur magnétique, maintenu à 30°C sur bain d'eau thermorégulé, on introduit sous agitation 230ml de microémulsion préparée comme indiqué dans l'exemple 1, et 10g d'α-terpinène (Composé I). On maintient sous agitation pendant 5 minutes environ pour obtenir une solution limpide.

A la solution ainsi obtenue, on ajoute ensuite 2ml d'eau oxygénée à 30% (110 volumes). On recommence 8 fois cette addition d'eau oxygénée toutes les 10 minutes de manière à ajouter au total 18ml d'eau oxygénée. Le temps total de réaction est de 90 minutes. L'évolution de la réaction est suivie par chromatographie liquide haute performance (CLHP).

On chasse le dichlorométhane et l'essentiel de l'eau et du butanol-1 sous vide à l'aide d'un évaporateur rotatif muni d'un bain à 40°C. Le résidu pâteux obtenu est extrait par deux fractions de 50ml de n-hexane. Le solide résiduel (dodécylsulfate de sodium, molybdate de sodium et tampon carbonate est récupéré sur verre fritté et peut être réutilisé pour une autre peroxydation.

L'hexane est éliminé par évaporation sous vide, et on obtient ainsi 10,4g d'ascaridol (Dérivé I') sous forme d'huile de couleur jaune à odeur caractéristique, que l'on purifie par distillation fractionnée sous pression réduite.

$Eb_{0,2}$ = 39-40°C. Rendement en produit pur: 8,6g (70%).

La structure de l'ascaridol est confirmée par spectre de RMN $^1H$. Les formules générales de l'α-terpinène (Composé I) et de l'ascaridol (Dérivé I') sont indiquées ci-dessous.

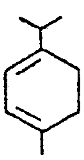
(I)

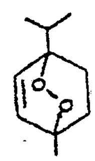
(I')

**EXEMPLES 2 - 8**

On procède comme dans l'exemple 1, en utilisant les composés organiques indiqués au tableau ci-après et en effectuant l'extraction par du dichlorométhane au lieu du n-hexane.

| Ex. N° | Composé | Poids (g) | Microém. ml | $H_2O_2$ ml | t min | Dérivé | Rdt % |
|---|---|---|---|---|---|---|---|
| 2 | (II) | 2 | 85 | 4 | 60 | (II') | 97 |
| 3 | (III) | 0,1 | 15 | 0,1 | 20 | (III') | 100 |
| 4 | (IV) | 0,1 | 2 | 0,1 | 40 | (IV') | 86 |
| 5 | (V) | 0,1 | 3 | 0,2 | 70 | (V') | 78 |
| 6 | (VI) | 0,1 | 2 | 0,1 | 30 | (VI') | 91 |
| 7 | (VII) | 0,1 | 3 | 0,3 | 90 | (VII') | 86 |
| 8 | (VIII) | 6,3 | 140 | 12 | 100 | (VIII') | 67 |

Les composés utilisés et les dérivés d'oxydation obtenus sont indiqués en détail ci-après:

Composé (II): diphényl-9,10 anthracène

Dérivé (II'): diphényl-9,10 dihydro-9,10 épidioxy-9,10 anthracène

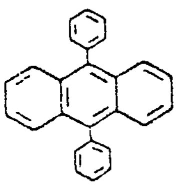

(II)

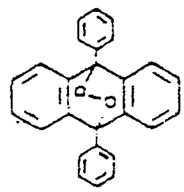

(II')

Composé (III):

rubrène

Dérivé (III'):

endoperoxyde de rubrène

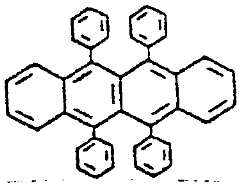

(III)

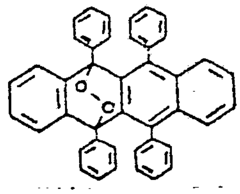

(III')

Composé (IV):

benzoate d'ergostérol

Dérivé (IV'):

endoperoxyde de benzoate d'ergostérol

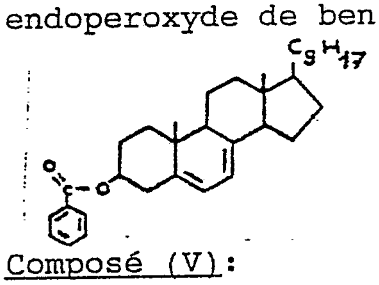

(IV)

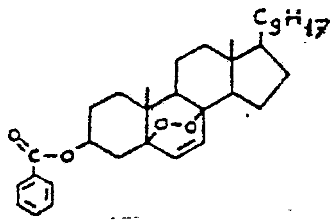

(IV')

Composé (V):

tétracyclone

Dérivé (V'):

α,α'-dibenzoylstilbène (Z)

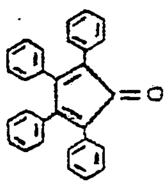

(V)

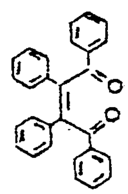

(V')

Composé (VI):

diphényl-1,3 isobenzofuranne

Dérivé (VI'):

dibenzoyl-1,2 benzène

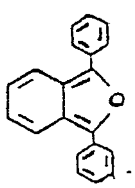

(VI)

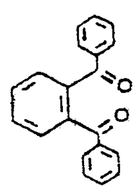

(VI')

<u>Composé (VII)</u>:

adamantylidène-adamantane

<u>Dérivé (VII')</u>:

bis(adamantylidène)-3,4 dioxétanne-1,2

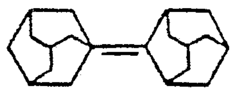

(VII)

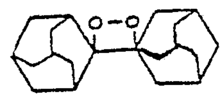

(VII')

<u>Composé (VIII)</u>:

ß-citronellol

<u>Dérivé (VIII')</u>:

diméthyl-3,7 hydroperoxy-6 octène-7 ol-1

diméthyl-3,7 hydroperoxy-7 octène-5 ol-1

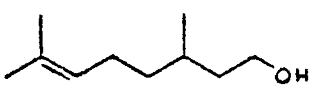

(VIII)

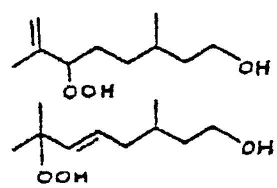

(VIII')

On obtient dans ce cas un mélange des deux dérivés (VIII') indiqués ci-dessus.

**EXEMPLE 10**

<u>Production d'une chimiluminescence</u>

On utilise la microémulsion préparée comme indiqué ci-dessus, mais à partir de 1g de molybdate de sodium, 44mg de carbonate de sodium, 35mg d'hydrogénocarbonate de sodium, 8,7g de dodécyl sulfate de sodium, 17,5g de butanol-1, 33ml de benzène et 67ml d'eau distillée.

75ml de cette microémulsion sont placés dans un Erlenmeyer, et on y dissout 0,14g de bis(N,N-diméthylamino-4' phényl)-2,3 dioxène-1,4.

On ajoute à la solution 10mg environ de fluoresceur constitué par la rhodamine 6G. Le récipient est plongé dans un bain d'eau thermorégulé à 40°C, et on y ajoute 0,75ml d'eau oxygénée 30%. On observe alors une émission lumineuse de couleur orange, qui se prolonge pendant environ deux minutes.

On réalise la même expérience avec le diphényl-9,10 anthracène et le bis(phényléthynyl)-9,10 anthracène comme fluoresceurs, et on observe l'émission d'une lumière bleue et verte, respectivement.

**Revendications**

1. Procédé d'oxydation de composés organiques par l'eau oxygénée en présence d'un catalyseur en milieu basique, caractérisé en ce qu'on fait agir l'eau oxygénée sur le composé organique en présence d'un catalyseur constitué par un molybdate ou un tungstate de métal alcalin, en microémulsion.

2. Procédé selon la revendication 1, caractérisé en ce que la microémulsion est obtenue par mélange d'une solution aqueuse du catalyseur, d'au moins un surfactif, et d'au moins un solvant organique.

3. Procédé selon la revendication 2, caractérisé en ce que le surfactif est un surfactif anionique choisi parmi les alkyl sulfates, les alkyléther sulfates, les alkyl sulfonates, les alkylaryl sulfonates, les alkylamide sulfonates, les alkyl sulfosuccinates, et les alkyl phosphates de métal alcalin.

4. Procédé selon la revendication 2, caractérisé en ce que le solvant organique est un hydrocarbure ou un hydrocarbure halogéné.

5. Procédé selon la revendication 4, caractérisé en ce que le solvant organique est choisi parmi le dichlorométhane, le dichloro-1,2 éthane, le chloroforme, le tétrachlorure de carbone, le trifluoro-1,1,2 trichloro-2,2,1 éthane (Fréon 113), le pentane, le n-hexane, le cyclohexane, le benzène, et le toluène.

6. Procédé selon la revendication 1, caractérisé en ce que la concentration en eau oxygénée libre dans la phase aqueuse est comprise entre 0,1M et 1M.

7. Procédé selon la revendication 1, caractérisé en ce que le catalyseur est mis en microémulsion en présence d'un tampon carbonate ou d'un tampon phosphate.

8 Procédé selon l'une quelconque des revendications précédentes, caractérisé en ce que le pH du milieu réactionnel est compris entre 8 et 12,5.

9. Procédé selon l'une quelconque des revendications précédentes, caractérisé en ce que la réaction s'effectue à une température comprise entre 10° C et 80° C.

10. Procédé selon la revendication 1, caractérisé en ce que le catalyseur est le molybdate de sodium ou le tungstate de sodium.

11. Procédé de production d'une chimiluminescence par réaction de cyclo-addition [2+2] d'oxygène singulet sur une double liaison ne comportant pas d'hydrogène allylique, caractérisé en ce qu'on fait agir de l'eau oxygénée en présence d'un catalyseur constitué par un molybdate ou un tungstate de métal alcalin en microémulsion, et d'un fluoresceur, sur le dioxène ou un dérivé du dioxène.

Office européen des brevets

# RAPPORT DE RECHERCHE EUROPEENNE

Numero de la demande

EP 88 40 0651

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | JOURNAL OF THE AMERICAN CHEMICAL SOCIETY, vol. 107, no. 21, 16 octobre 1985, pages 5844-5849, American Chemical Society; J.M. AUBRY: "Search for singlet oxygen in the decomposition of hydrogen peroxide by mineral compounds in aqueous solutions" * Tableaux I,II,IV * --- | 1-10 | C 07 B 41/00<br>C 09 K 11/07 //<br>C 07 D 493/08<br>C 07 J 71/00<br>C 07 C 49/796<br>C 07 C 49/784<br>C 07 D 321/00<br>C 07 C 179/025<br>C 07 C 45/28<br>C 07 C 178/00 |
| A,D | GB-A-1 324 763 (CONTINENTAL OIL) * Revendications, tableau III * ----- | 1-10 | |

DOMAINES TECHNIQUES RECHERCHES (Int. Cl.4)

C 07 B 41/00
C 07 C 45/00
C 07 C 178/00
C 07 C 179/00
C 07 D 493/00

Le présent rapport a été établi pour toutes les revendications

| Lieu de la recherche | Date d'achèvement de la recherche | Examinateur |
|---|---|---|
| LA HAYE | 01-07-1988 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................
& : membre de la même famille, document correspondant

EPO FORM 1503 03.82 (P0402)